# EUROPÄISCHE PATENTSCHRIFT

(11) **EP 2 756 765 B1**
(45) Veröffentlichungstag und Bekanntmachung des Hinweises auf die Patenterteilung: **27.03.2019**
(21) Anmeldenummer: 13151578.5
(22) Anmeldetag: 17.01.2013
(51) Int. Cl.: A23F 3/40, A23F 5/46, A61K 9/00, A61K 9/68, A61K 9/19, A61K 9/48, A23L 2/52, A61K 47/22, A61K 31/341, A61K 31/353, A61K 31/365, A23L 27/00, A23L 33/10, G01N 33/50, C07K 14/705

(54) **Pharmazeutische Zubereitungen**
Pharmaceutical compositions
Compositions pharmaceutiques

(43) Veröffentlichungstag der Anmeldung: 23.07.2014
(73) Patentinhaber: Symrise AG, 37603 Holzminden (DE)
(72) Erfinder: Somoza, Veronika, 3400 Weidling (AT); Liszt, Kathrin Ingrid, 7471 Rechnitz (AT); Köck, Elke, 8820 Neumarkt (AT); Ley, Jakob, 37603 Holzminden (DE); Widder, Sabine, 37603 Holzminden (DE)
(74) Vertreter: Fabry, Bernd

(56) Entgegenhaltungen:
- EP-A2- 0 534 024
- EP-A2- 1 258 200
- US-A- 4 865 847
- M. T. MONFORTE ET AL: "Protective Effect of Calamintha officinalis Moench Leaves against Alcohol-induced Gastric Mucosa Injury in Rats. Macroscopic, Histologic and Phytochemical Analysis", PHYTOTHERAPY RESEARCH, Bd. 26, Nr. 6, 10. Juni 2012 (2012-06-10), Seiten 839-844, XP055062854, ISSN: 0951-418X, DOI: 10.1002/ptr.3647
- TRAUTMANN M ET AL: "Aspirin-like drugs, ethanol-induced rat gastric injury and mueosal eicosanoid release", EUROPEAN JOURNAL OF PHARMACOLOGY, ELSEVIER SCIENCE, NL, Bd. 201, Nr. 1, 16. August 1991 (1991-08-16), Seiten 53-58, XP023830062, ISSN: 0014-2999, DOI: 10.1016/0014-2999(91)90322-H [gefunden am 1991-08-16]
- MAGOUS R ET AL: "Leukotrienes stimulate acid secretion from isolated gastric parietal cells", BIOCHEMICAL AND BIOPHYSICAL RESEARCH COMMUNICATIONS, ACADEMIC PRESS INC. ORLANDO, FL, US, Bd. 114, Nr. 3, 12. August 1983 (1983-08-12), Seiten 897-900, XP024846252, ISSN: 0006-291X, DOI: 10.1016/0006-291X(83)90644-7 [gefunden am 1983-08-12]
- ESWARAN M B ET AL: "Gastroprotective activity of Cinnamomum tamala leaves on experimental gastric ulcers in rats", JOURNAL OF ETHNOPHARMACOLOGY, ELSEVIER SCIENTIFIC PUBLISHERS LTD, IE, Bd. 128, Nr. 2, 24. März 2010 (2010-03-24) , Seiten 537-540, XP026939854, ISSN: 0378-8741, DOI: 10.1016/J.JEP.2010.01.036 [gefunden am 2010-01-18]
- GAURAV KAITHWAS ET AL: "Evaluation of antiulcer and antisecretory potential of Linum usitatissimum fixed oil and possible mechanism of action", INFLAMMOPHARMACOLOGY, Bd. 18, Nr. 3, 1. Juni 2010 (2010-06-01), Seiten 137-145, XP055076413, ISSN: 0925-4692, DOI: 10.1007/s10787-010-0037-5
- GURBUZ, I., ERDEMOGLU, N., YESILADA E., SENER, B.: "Anti-ulcerogenic lignans from Taxus baccata L.", ZEITSCHRIFT FÜR NATURFORSCHUNG, Bd. 59c, 31. Dezember 2004 (2004-12-31), Seiten 233-236, XP002711914, Tübingen
- J.K. RAMAGE, A. DENTON, J.G. WILLIAMS: "Inhibition of food stimulated acid secretion by misoprostol, an orally active synthetic E1 ananolgue prostaglandin", BR. J. CLIN. PHARMAC., Bd. 19, 31. Dezember 1985 (1985-12-31), Seiten 9-12, XP002711915,

## Beschreibung

### GEBIET DER ERFINDUNG

Die Erfindung befindet sich auf dem Gebiet der Pharmazie und Nahrungsmittel und betrifft Zubereitungen, die Wirkstoffe gegen die durch Nahrungsmittelbestandteile induzierte Sekretion von Magensäure enthalten.

### STAND DER TECHNIK

Die Stimulation der Magensäuresekretion stellt einen wichtigen Mechanismus zur Einleitung der Verdauung von Nahrung, insbesondere proteinreicher Nahrung dar. Es kann durchaus positiv für die Verdauung sein, die Sekretion kurzfristig maßvoll zusätzlich anzuregen. Wird Magensäure aber durch externe Stimuli übermäßig sekretiert und somit der pH im Magen zu stark gesenkt, so führt das akut in der Regel z.B. zu Unwohlsein oder saurem Aufstoßen. Wenn dieser Zustand länger anhält oder chronisch die Magensäuresekretion stark angeregt wird, so können entzündliche Zustände der Magenschleimhaut sowie der Speiseröhre induziert werden, die ihrerseits wiederum als Spätfolge Geschwüre und im schlimmsten Fall auch bösartige Gewebeveränderungen bis zum Magenkrebs und Speiseröhrenkrebs auslösen.

Die Magensäuresekretion wird neben den allgemeinen Stimuli wie Nahrungsaufnahme, Hungerzustände etc. auch durch diverse niedermolekulare Nahrungsbestandteile induziert. Z.B. wird von Liszt et al. in J. AGRIC FOOD CHEM. 60, 7022-7030 (2012**)** beschrieben, dass die in Weißwein verstärkt vorkommenden Fruchtsäuren Äpfelsäure und Bernsteinsäure einen wesentlichen Beitrag zur übermäßigen Säuresekretion liefern, die im Falle der Weinsäure aber durch Alkohol wieder gemildert werden kann. Auch können Hopfeninhaltsstoffe z.B. über Bierkonsum zu einer Ausschüttung von Protonen in den Mageninhalt induzieren (vgl. Walker et al. in J. AGRIC FOOD CHEM. 60, 1405-1412 (2012**),** wobei eine Tendenz der relativen Bitterkeit und der Aktivität auf die Protonensekretion zu erkennen ist. Auch Kaffee und insbesondere das darin enthaltende Coffein ist in der Lage, Magensäuresekretion signifikant zu stimulieren, wie von Rubach et al., in MOL. NUTR. FOOD RES. 56, 325-335 (2012**)** gezeigt werden konnte.

Aus dem Aufsatz von Monforte et al. mit dem Titel "Protective effect of Calamintha officinalis Moench leaves against alcohol-induced gastric mucosa injury in rats" in PHY-TOTHERAPY RES 26(6), S. 839-844 (2012**)** ist bekannt, dass der Mechanismus der Ulkusbildung bei Ethanolgabe unter anderem in der Induktion der Protonensekretion gesehen wird. Die gastroprotektive Wirkung des Extraktes wird dabei auf die verschiedenen Bestandteile, insbesondere auf die Anwesenheit von Eriodyctyol zurückgeführt, wobei eine inhibitorische Wirkung dieser Stoffe auf die H⁺K⁺ ATPase unterstellt wird.

Klassisch verwendet man zwei große Gruppen von Wirkstoffen zur Reduktion der oben beschriebenen Magensäuresekretion: Neutralisation der Magensäure mit Hilfe von basischen Materialien wie Natriumhydrogencarbonat, Calciumcarbonat, basischen Aluminium- oder Magnesiumhydroxiden etc. auf der einen Seite zu Anhebung des pH-Wertes; andererseits Senkung der Magensäuresekretion durch direkte Blockierung der auf den sekretorischen Zellen (Parietalzellen) zu findenden Acetylcholin-Rezeptoren (M₃-Typ) durch Wirkstoffe wie Pirenzepin oder öfter H₂-Histaminrezeptoren z.B. durch Wirkstoffe wie Cimetidin, Ranitidin oder Famotidin; als drittes Wirkprinzip wird direkt die ATP-getriebene Protonenpumpe der Parietalzellen moduliert (Opremazol).

Der Mechanismus der stimulierenden Wirksamkeit der oben genannten Nahrungsmittelbestandteile ist in der Regel nicht klar. Daher ist auch nicht vorhersehbar, welche für oder in Nahrungsmittel verwendbare niedermolekulare Stoffe (außer basischen Stoffen durch ihre Pufferwirkung) gegebenenfalls auch die Wirkung umkehren können und somit die übermäßige Sekretion verringern können.

Aufgabe der Erfindung war es daher, für oder in Nahrungsmittel verwendbare niedermolekulare Stoffe, bevorzugt natürliche Stoffe zu finden, die sich gegenüber den bekannten Wirkstoffen durch eine bessere Verträglichkeit und höhere Leistungsfähigkeit auszeichnen und die durch die oben genannten Nahrungsmittelbestandteile induzierte Säuresekretion mindern oder sogar vollständig verhindern können.

### BESCHREIBUNG DER ERFINDUNG

Ein erster Gegenstand der Erfindung betrifft pharmazeutische Zubereitungen, enthaltend
bitter-maskierend wirkende Hydroxyflavanonen ausgewählt aus der Gruppe bestehend aus Eriodictyol-7-methylether, Homoeriodictyol, und deren Natrium-, Kalium-, Calcium-, Magnesium- oder Zinksalzen (Komponente a)
zur Verwendung zur Verminderung und/oder Inhibierung der von Nahrungsmittelbestandteilen (Komponente b) induzierten Magensäuresekretion,
ausgewählt aus der Gruppe, die gebildet wird von Xanthin, Fruchtsäuren, phenolischen Glykosiden, Flavonglykosiden, Dihydrochalconglykosiden, hydrolysierbaren und nicht-hydrolysierbaren Tanninen und deren Oligomeren, terpenoiden oder iridoiden Bitterstoffen und Triterpenglykosiden sowie deren Gemischen.

Überraschenderweise wurde gefunden, dass Stoffe, die in der Lage sind die Bitterkeit vieler Nahrungsmittel zu maskieren, auch gleichzeitig die durch diese Stoffe induzierte Sekretion von Magensäure vermindern oder sogar inhibieren. Insbesondere wurde festgestellt, dass die Stoffe der Gruppe (a), wie z.B. Homoeriodictyol, Eriodictyol, Matairesinol, Lariciresinol oder Enterolacton die magensäurestimulierende Wirkung der Stoffe der Gruppe (b), wie z.B. Coffein oder Theobromin, in Kombination wieder senken oder gänzlich blockieren können.

Dies ist umso erstaunlicher, als man bislang davon ausgegangen ist, dass die Maskierungsstoffe lediglich an bestimmte Geschmacksrezeptoren andocken und nicht auch noch eine pharmakologische Wirkung besitzen. Die Verwendung der Maskierungsstoffe für den angegebenen Zweck hat dabei den weiteren Vorteil, dass sie sich bereits als ausgesprochen bekömmlich erwiesen haben.

**BITTER-MASKIERENDE STOFFE** Die bitter-maskierende Stoffe, die die Komponente (a) bilden, stellen wie erwähnt Hydroxyflavanone dar, insbesondere Eriodictyol, Eriodictyol-7-methylether, Homoeriodictyol, und deren Natrium-, Kalium-, Calcium-, Magnesium- oder Zinksalzen, speziell solches wie sie in der Europäischen Patentanmeldung EP 1258200 A2 beschrieben werden.

### SEKRETION VON MAGENSÄURE INDUZIERENDE NAHRUNGSMITTELBESTANDTEILE

Diese Stoffe, die die Komponente (b) umfassen, sind ausgewählt aus der Gruppe, die gebildet wird von:
- ***Xanthine,*** insbesondere Coffein, Theobromin und Theophyllin;
- ***Fruchtsäuren,*** insbesondere Weinsäure, Traubensäure, Äpfelsäure und Bernsteinsäure;
- ***phenolische Glycoside,*** insbesondere Salicin und Arbutin;
- ***Flavanonglycoside,*** insbesondere Neohesperedin, Eriocitrin, Neoeriocitrin, Narirutin und Naringin;
- ***Dihydrochalconglycoside,*** insbesondere Phloridzin, Trilobatin;
- ***hydrolisierbare Tannine,*** insbesondere Gallus- oder Ellagsäureester von Kohlenhydraten, z.B. Pentagalloylglucose;
- ***nicht-hydrolisierbare Tannine,*** insbesondere galloylierte Catechine oder Epicatechine und deren Oligomeren, z.B. Proanthyocyanidine oder Procyanidine, Thearubigenin), Flavone und deren Glycoside, insbesondere Quercetin, Quercitrin, Rutin, Taxifolin, Myricetin, Myrictrin;
- ***terpenoide Bitterstoffe,*** insbesondere Limonin, Nomilin, Lupolone und Humolone;
- ***Triterpenglycoside,*** insbesondere Stevioside, Rubusosid, Steviosid, Rebaudiosid A, Rebaudioside C, Glycyrrhizin (Glycyrrhizinsäure) und Glycyrrhetinsäure); und
- ***iridoide Bitterstoffe*** wie z.B. Oleuropein
sowie deren Gemische.

### BEVORZUGTE AUSFÜHRUNGSFORMEN

Die stärkste Verminderung bzw. eine vollständige Inhibierung der induzierten Magensäuresekretion wurde in Zubereitungen beobachtet, die als Komponente (b) Coffein und/oder Theobromin enthielten und denen man vorzugsweise Homoeriodictyol 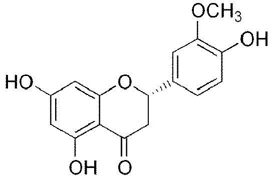 zusetzt.

Grundsätzlich können die therapeutischen Zubereitungen sowohl die Stoffe, die die Komponente (a) als auch (b) bilden, jeweils in Mengen von etwa 0,1 mg/kg (entsprechend 0,1 ppm) bis etwa 1 Gew.-% enthalten. Vorzugsweise sind die Stoffe jedoch jeweils in Mengen von etwa 0,5 bis etwa 1.000 mg/kg (entsprechend etwa 0,5 bis etwa 1.000 ppm), bevorzugt von etwa 1 bis etwa 500 mg/kg, weiter bevorzugt von etwa 3 bis etwa 300 mg/kg, besonders bevorzugt von etwa 5 bis etwa 200 mg/kg und am meisten bevorzugt von etwa 10 bis 100 mg/kg enthalten.

Die erfindungsgemäßen Zubereitungen können die Stoffe, die die Komponenten (a) und (b) bilden, im Gewichtsverhältnis von etwa 1:100 bis etwa 1:1, vorzugsweise 1:90 bis etwa 1:2 und insbesondere von etwa 1:5 bis etwa 1:10 enthalten.

### ORALE ZUBEREITUNGEN

Ein weiterer Gegenstand der vorliegenden Erfindung betrifft speziell der Ernährung oder dem Genuss dienende Zubereitungen zur oralen Aufnahme, enthaltend wie vorstehend bereits ausführlich erläutert
(a) bitter-maskierend wirkende Hydroxyflavanone ausgewählt aus der Gruppe bestehend aus Eriodictyol-7-methylether, Homoeriodictyol, und deren Natrium-, Kalium-, Calcium-, Magnesium- oder Zinksalzen (Komponente a), und
(b) Stoffe die die Sekretion von Magensäure induzieren, ausgewählt aus der Gruppe, die gebildet wird von Xanthin, Fruchtsäuren, phenolischen Glykosiden, Flavonglykosiden, Dihydrochalconglykosiden, hydrolysierbaren und nicht-hydrolysierbaren Tanninen und deren Oligomeren, terpenoiden oder iridoiden Bitterstoffen und Triterpenglykosiden sowie deren Gemischen (Komponente b),

wobei sich die Zubereitungen dadurch auszeichnen, dass sie die Komponente (a) und (b) jeweils in Mengen von 3 bis 300 ppm enthalten.

Der Ernährung oder dem Genuss dienende (d.h. zum Verzehr geeignete) Zubereitungen sind im Rahmen dieser Erfindung z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder vollständig hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayon-naise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Die oralen Zubereitungen im Sinne der Erfindung können auch als Halbfertigware zur Herstellung weiterer oral konsumierbarer Zubereitungen dienen. Die Zubereitungen im Sinne der Erfindung können auch in Form von Kapseln, Tabletten (oral und/oder magenauflösende Tabletten), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Bevorzugt werden dabei einer oder mehrerer der Stoffe der Gruppe (a) in eine oral konsumierbare Zubereitung, insbesondere in Form von schluckbaren Zubereitungen (z.B. Getränke, Dispersionen, Emulsionen, Pasten, im Mund oder Magen auflösbare Kapseln, Tabletten, Komprimate, Weichkaramelle, Dragees, Granulaten, Pellets, Fruchtgummis) oder ausreichende Konzentrationen Stoffe der Gruppe (a) freisetzende nicht zum Schlucken intendierte Zubereitungen (z.B. Kaugummi, Hartkaramelle) in einer wirksamen Konzentration eingearbeitet.

Die erfindungsgemäß einzusetzenden Stoffe der Gruppe (a) weisen in erfindungsgemäßen zum Verzehr geeignete Zubereitungen, insbesondere in den erfindungsgemäß bevorzugt einzusetzenden Konzentrationen, keinen nennenswerten Eigengeschmack auf, insbesondere zeigen sie in den (bevorzugten bzw. besonders bevorzugten) Einsatzkonzentrationen keine unangenehmen oder störenden Geschmacksnoten. Dabei sind die Stoffe der Gruppe (a) jeweils alleine und/oder in Mischung in der Lage, die Bitterkeit eines oder mehrerer der Stoffe der Gruppe (b) zu reduzieren oder ganz zu blockieren.

Der oder die Stoffe der Gruppe (a) sowie erfindungsgemäßer Zubereitungen, enthaltend den oder die Stoffe der Gruppe (a), können zeitlich vor, während oder nach der oralen Einnahme von dem oder den Stoffen der Gruppe (b) oder Zubereitungen, enthaltend den oder die Stoffe der Gruppe (b) oral zugeführt werden.

Besonders vorteilhaft ist dabei die bekannte Tatsache, dass die Stoffe der Gruppe (a) regelmäßig in der Lage sind, den bitteren (unangenehmen) Geschmack der Stoffe der Gruppe (b) teilweise oder ganz zu reduzieren und somit die Akzeptanz für die Zubereitungen deutlich steigern können. Dies ist insbesondere der Fall, wenn die Stoffe der Gruppe (b) zeitlich gleichzeitig mit Stoffen der Gruppe (a) appliziert werden.

Bei nachfolgender oraler Einnahme des oder der Stoffe der Gruppe (a) sind Zubereitungsformen bevorzugt, die den oder die Stoffe der Gruppe (a) in einer Form enthalten, die eine verzögerte Freisetzung bewirken.

Alternativ zur gleichzeitigen oralen Einnahme können sind auch Zubereitungen geeignet, die
einen oder mehrere der unangenehm schmeckende und magensäurestimulierende Stoffe der Gruppe (b) in einer in Bezug auf die Magensäurestimulation wirksamen Menge aufweisen und gleichzeitig einen oder mehrere der Stoffe der Gruppe (a) in einer in Bezug auf die Magensäurestimulation wirksamen Menge enthalten.

Grundsätzlich können die oralen Zubereitungen - seien es Fertig- oder Halbfertigprodukte - sowohl die Stoffe, die die Komponente (a) als auch (b) bilden, jeweils in Mengen von etwa 0,1 mg/kg (entsprechend 0,1 ppm) bis etwa 1 Gew.-% enthalten. Vorzugsweise sind die Stoffe jedoch jeweils in Mengen von etwa 0,5 bis etwa 1000 mg/kg (entsprechend etwa 0,5 bis etwa 1000 ppm), bevorzugt von etwa 1 bis etwa 500 mg/kg, weiter bevorzugt von etwa 3 bis etwa 300 mg/kg, besonders bevorzugt von etwa 5 bis etwa 200 mg/kg und am meisten bevorzugt von etwa 10 bis 100 mg/kg enthalten.

Die erfindungsgemäßen Zubereitungen können die Stoffe, die die Komponenten (a) und (b) bilden, im Gewichtsverhältnis von etwa 1:100 bis etwa 1:1, vorzugsweise 1:90 bis etwa 1:2 und insbesondere von etwa 1:5 bis etwa 1:10 enthalten.

### GEWERBLICHE ANWENDBARKEIT

Eine bevorzugte Methode zur Ermittlung der gewünschten Wirkung von Stoffen der Gruppe (a) zur Senkung der Wirkung der Stoffe der Gruppe (b) besteht in der Messung des intrazellulären pH-Wertes von HGT-1-Zellkulturen (human gastric tumor cell line) nach Behandlung mit den Teststoffen wie in Liszt, K. I.; Walker, J.; Somoza, V.. J. Agric Food Chem. 2012, 60, (28), 7022-7030 beschrieben. Bisher ist auf der Zelloberfläche von Magensäuresekretierenden Zellen nur der Somatostatin-Rezeptor (SSTR2) als Target für die Reduktion der Sekretion beschrieben worden; dieser Rezeptor ist auch in der HGT-1 beschrieben worden. Bisher sind diese Zellen aber immer nur in Kontakt mit potentiellen SSTR2-Regulatoren gebracht worden, die gemeinsame Verabreichung von bitteren Agonisten und potentiellen Antagonisten ist bisher nicht beschrieben worden.

### BEISPIELE

### ZELLMODELL

Als Zellmodell werden humane Parietalzellen, auch als human gastric tumor cell line (HGT-1) bekannt, verwendet. Diese wurden von Dr. C. Laboisse (Laboratory of Pathological Anatomy, Nantes, France) zur Verfügung gestellt. Die Zellen werden unter den Standardbedingungen 37 °C, 5 % CO₂ in DMEM (Dulbecco's Modified Eagle's Medium) mit 4 g/L Glucose, 10 % FBS, 2 % L-Glutamin und 1 % Penicillin/Streptomycin kultiviert. Für die Messung der intrazellulären Protonenkonzentration werden die Zellen mit Trypsin/EDTA behandelt, die Viabilität mittels Tryptanblaufärbung bestimmt und 100 000 Zellen/well in schwarzen 96-well Platten ausgesät.

**BEISPIEL 1**

### Bestimmung des intrazellulären pH-Wertes von HGT-1-Zellkulturen in Gegenwart von Stoffen der Gruppe (a) und Stoffen der Gruppe (b)

Zur Messung des intrazellularen pH-Wertes wird der Farbstoff 1,5 Carboxy-Seminaphto-rhodafluor Acetoxymethylester (SNARF-1-AM) verwendet. Die Zellen in den 96-well Platten werden einmal mit Krebs-Hepes-Puffer (KRHP) gewaschen und mit dem Farbstoff in der Konzentration von 3 µM gelöst in KRHP für 30 min bei 37 °C und 5 % CO₂ inkubiert. Danach werden die Zellen zwei Mal mit KRHP gewaschen und mit Stoffen der Gruppe (b), beispielsweise 3 mM Coffein beziehungsweise 0,3 mM Theobromin alleine oder in Kombination mit Stoffen der Gruppe (a), beispielsweise Homoeriodictyol (HED) oder Eriodictyol oder Matairesinol, Lariciresinol bzw. Enterolacton in unterschiedlichen Konzentrationen in phenolrotfreiem DMEM im Volumen von 100 µL aufgetragen; als weiteres Kontrollexperiment werden die oben genannten Stoffe der Gruppe (a) alleine geprüft. Homoeriodictyol wird in doppelt destilliertem Wasser gelöst während Eriodictyol, Matairesinol, Lariciresinol bzw. Enterolacton in Ethanol (EtOH) gelöst werden. Die finale Konzentration an Lösungsmittel, die den Zellen zugeführt wird, beträgt maximal 1 %. Der Fluoreszenzfarbstoff wird bei der Wellenlänge von 488 nm angeregt und die Emission bei 580 nm und 640 nm gemessen. Das Verhältnis der Fluoreszenzwerte von 580 nm zu 640 nm wird aufgetragen im Vergleich zur Kalibrierkurve, woraus der pH-Wert ermittelt werden kann. Für die Kalibrierkurve werden die Zellen mit einem Kaliumpuffer unterschiedlicher pH-Werte von 7,2 - 8,2 pH und 2 µM Nigericin behandelt. Nigericin equilibriert den intrazellulären und extrazellulären pH-Wert, sodass der intrazelluläre pH-Wert definiert werden kann. Die intrazelluläre H⁺ - Konzentration wird aus dem intrazellulären pH-Wert ermittelt. Der intrazelluläre Protonenindex (IPX) wird kalkuliert durch log2 Transformation des Verhältnisses zwischen behandelten Zellen und unbehandelten Zellen (Kontrolle). Die hier dargestellten Ergebnisse werden als prozentuale Veränderung im Vergleich zu nicht-behandelten Kontrollzellen angegeben (vgl. Malte Rubach, R. L., Elisabeth Seebach, Mark M. Somoza, Thomas Hofmann; Somoza, a. V., Mol Nutr Food Res. in press, 2011; Rubach, M.; Lang, R.; Hofmann, T.; Somoza, V., Ann N Y Acad Sci 2008, 1126, 310-4; Rubach, M.; Lang, R.; Skupin, C.; Hofmann, T.; Somoza, V., J Agric Food Chem 2010, 58, 4153-61; Weiss, C.; Rubach, M.; Lang, R.; Seebach, E.; Blumberg, S.; Frank, O.; Hofmann, T.; Somoza, V., J Agric Food Chem 2010, 58, 1976-85; Liszt, K. I.; Walker, J.; Somoza, V., J Agric Food Chem 2012; Walker, J.; Hell, J.; Liszt, K. I.; Dresel, M.; Pignitter, M.; Hofmann, T.; Somoza, V., J Agric Food Chem 2012, 60, 1405-12). Die Anzahl der angegebenen Replikate beziehen sich auf technische Replikate (tr) oder auf die Anzahl an Gesamtreplikaten (n), die sich aus der Anzahl der technischen Replikate multipliziert mit der Anzahl an biologischen Replikaten ergibt.

In der folgenden **Tabelle 1A** ist die prozentuale Steigerung der Protonensekretion in HGT-1 Zellen im Vergleich zu unbehandelten Kontrollen nach 10 minütiger Stimulierung durch DMEM oder DMEM mit ETOH 1% oder 1mM Histamin oder 3 mM Coffein mit und ohne EtOH 1% angegeben. Die Daten sind dargestellt als Mittelwert und mittlere Standardabweichung, n=21-37, tr=6. Statistik: one-way Anova mit post-hoc Test nach Dunn's. Signifikante Unterschiede (p < 0,05) werden durch Buchstaben angegeben.

**Tabelle 1A**

| Vergleichsbeispiel Steigerung der Sekretion durch Stoffe der Gruppe (b) | | |
|---|---|---|
| **Testsubstanz** | **T/C [%]** | **SEM** |
| Kontrolle (DMEM) | 0.150 | 1.00 a |
| EtOH 1% | 14.44 | 2.61 b |
| 1 mM Histamin | 27.42 | 3.17 b |
| 3 mM Coffein | 48.87 | 3.64 d |
| 3 mM Coffein + EtOH 1% | 44.92 | 3.51 d |

In der folgenden **Tabelle 1B** ist die prozentuale Steigerung der Protonensekretion in HGT-1 Zellen im Vergleich zu unbehandelten Kontrollen nach 10 minütiger Stimulierung durch DMEM oder DMEM mit ETOH 1% oder 1mM Histamin oder 0.3 mM Theobromin mit und ohne EtOH 1% angegeben. Die Daten sind dargestellt als Mittelwert und mittlere Standardabweichung, n=21-37, tr=6. Statistik: one-way Anova mit post-hoc Test nach Dunn's. Signifikante Unterschiede (p < 0,05) werden durch Buchstaben angegeben.

**Tabelle 1B**

| Vergleichsbeispiel Steigerung der Sekretion durch Stoffe der Gruppe (b) | | |
|---|---|---|
| **Testsubstanz** | **T/C [%]** | **SEM** |
| DMEM (Kontrolle) | 0.15 | 1.00 a |
| EtOH 1% | 14.44 | 2.61 b |
| 1 mM Histamin | 27.42 | 3.17 b,c |
| 0.3 mM Theobromin | 17.51 | 2.65 b |
| 0.3 mM Theobromin + EtOH 1% | 32.11 | 2.49 c,d |

In der folgenden **Tabelle 2** ist die prozentuale Steigerung der Protonensekretion in HGT-1 Zellen im Vergleich zu unbehandelten Kontrollen nach 10 minütiger Stimulierung durch DMEM (Kontrolle) oder Homoeriodictyol (HED) in unterschiedlichen Konzentrationen angegeben. Die Daten sind dargestellt als Mittelwert und mittlere Standardabweichung, n=4, tr=6. Statistik: one-way Anova mit post-hoc Test nach Dunn's. Signifikante Unterschiede (p < 0,05) werden durch Buchstaben angegeben.

**Tabelle 2**

| Beeinflussung der Sekretion durch Stoffe der Gruppe (a) | | |
|---|---|---|
| **Testsubstanz** | **T/C [%]** | **SEM** |
| DMEM (Kontrolle) | -1.43 | 2.01 a |
| 0.003 mM HED | -8.04 | 3.88 a |
| 0.03 mM HED | 12.70 | 4.02 a |
| 0.3 mM HED | -3.66 | 5.24 a |

### ANWENDUNGSBEISPIEL 1

### Sprühgetrocknete Zubereitung als Halbfertigware zur Herstellung von Fertigwaren

Das Trinkwasser wird in einem Behälter vorgelegt und das Maltodextrin und das Gummi Arabicum darin gelöst. Anschließend werden die Stoffe der Gruppe (a) mit einem Turrax in die Trägerstofflösung emulgiert. Die Temperatur der Sprühlösung sollte 30°C nicht überschreiten. Das Gemisch wird dann sprühgetrocknet (Solltemperatur Eingang: 185 - 195°C, Solltemperatur Ausgang: 70 - 75°C). Die sprühgetrocknete Halbfertigware enthält ca. 18 - 22 % der Verbindungen der Gruppe (a).

**Tabelle 3**

| Zusammensetzung Halbfertigware - Mengenangaben als Gew.-% | | |
|---|---|---|
| **Zubereitung** | **A** | **B** |
| Trinkwasser | 60,8 | 60,8 |
| Maltodextrin aus Weizen | 24,3 | 24,3 |
| Gummi Arabicum | 6,1 | 6,1 |
| Homoeriodictyol | 8,8 | 4,4 |

### ANWENDUNGBEISPIEL 2

### Trinkbare Eisteezubereitung

Die Verbindungen der Gruppe (a) wurden jeweils 10 % in Ethanol vorgelöst. Schwarztee-Extrakt wurde in Wasser gelöst und zusammen mit Zucker, einer Aromazubereitung (Pfirsichgeschmack), sowie den ethanolischen Lösungen der Verbindungen der Gruppe (a) in einem Becherglas verrührt.

**Tabelle 4**

| Zusammensetzung Eistee - Mengenangaben als Gew.-% | |
|---|---|
| **Zubereitung** | **A** |
| Schwarztee-Extrakt | 1,4 |
| Wasser | 89,5 |
| Aromazubereitung (Typ Pfirsich) | 0,65 |
| Zucker | 7,0 |
| Zitronensäure (kristallin) | 1,2 |
| Ascorbinsäure | 0,2 |
| Homoeriodictyol in Ethanol (10 %) | 0,05 |

### ANWENDUNGSBEISPIEL 3

### Gelatinekapseln ohne Stoffe der Gruppe (b) zum Direktverzehr nach Applikation von Stoffen der Gruppe (b)

Die zum Direktverzehr geeigneten Gelatinekapseln wurden gemäß WO 2004/050069 hergestellt und hatten einen Durchmesser von 5 mm, das Gewichtsverhältnis von Kernmaterial zu Hüllmaterial lag bei 90:10. Die Kapseln öffneten sich im Mund innerhalb von weniger als 10 Sekunden und lösten sich vollständig innerhalb von weniger als 50 Sekunden auf.

**] Tabelle 5**

| Zusammensetzung Gelatinekapsel - Mengenangaben als Gew.-% | |
|---|---|
| **Inhaltsstoff** | **A** |
| **Gelatinehülle:** | |
| Glycerin | 2,014 |
| Gelatine 240 Bloom | 7,91 |
| Sucralose | 0,065 |
| Allura Rot | 0,006 |
| Brillantblau | 0,005 |

| **Kernzusammensetzung:** | |
|---|---|
| Pflanzenöl-Triglycerid (Kokosöl - Fraktion) | 79,49 |
| Orangen-Aroma, enthaltend 1 Gew.-% Homoeriodictyol bezogen auf das Gesamtgewicht des Aromas | 10,0 |
| Rebaudiosid A 98 % | 0,05 |
| 2-Hydroxypropylmenthylcarbonat | 0,33 |
| Vanillin | 0,07 |

### ANWENDUNGSBEISPIEL 4

### Kaugummi ohne Stoff der Gruppe (b)

Teile A bis D werden gemischt und intensiv geknetet. Die Rohmasse kann z.B. in Form von dünnen Streifen zu verzehrfertigen Kaugummis verarbeitet werden.

**] Tabelle 6**

| Zusammensetzung Kaugummi - Mengenangaben als Gew.-% | | |
|---|---|---|
| **Teil** | **Zubereitung** | **A** |
| A | Kaugummibase, Company "Jagum T" | 30,00 |
| B | Sorbit, pulverisiert | 39,00 |
| | Isomalt® (Palatinit GmbH) | 9,50 |
| | Xylit | 2,00 |
| | Mannit® | 3,00 |
| | Aspartam® | 0,10 |
| | Acesulfam® K | 0,10 |
| | Emulgum® (Colloides Naturels, Inc.) | 0,30 |
| C | Sorbitol, 70% | 14,00 |
| | Glycerin | 1,00 |
| | Tutti-Frutti-Aroma, enthaltend 1% Homoeriodictyol, bezogen auf das Gesamtgewicht des Aromas | 2,00 |

### ANWENDUNGSBEISPIEL 5

### Verwendung in einem löslichen Cappuccino-Getränk

Die angegebenen Rohstoffe werden vermischt. Jeweils 12,5 g des hergestellten Instant-Cappuccino-Pulvers werden in 150 ml heißem Wasser gelöst.

**Tabelle 7**

| Zusammensetzung Cappuccino-Getränk - Mengenangaben als Gew.-% | |
|---|---|
| **Zubereitung** | **A** |
| Kaffee-Extrakt, sprühgetrocknet | 16,0 |
| Zucker | 25,3 |
| Fettpulver | 18,2 |
| Kaffeeweißer, schäumend | 28,0 |
| Hydrokolloide/ Emulgatoren | 1,8 |
| Lactose | 4,7 |
| Halbfertigware B aus Anwendungsbeispiel 1 | 6,0 |

### ANWENDUNGBEISPIEL 6

### Teegetränk

Der Tee und die Halbfertigware werden gemischt und in Teebeutel aus Filterpapier abgepackt. Zur Anwendung wird ein Teebeutel in 100 - 250 ml kochendes Wasser aufgegossen und 2 - 5 min ziehen gelassen.

**Tabelle 8**

| Zusammensetzung Teegetränk - Mengenangaben als Gew.-% | |
|---|---|
| **Zubereitung** | **A** |
| grüner Tee, China, Blattware | 91,90 |
| Halbfertigware B aus Anwendungsbeispiel 2 | 8,0 |
| Aroma (Typ Zitrone) | 0,1 |

### ANWENDUNGSBEISPIEL 7

### Verwendung in einer bitteren Schokolade

Eine bittere Schokolade wurde aus folgenden Rohstoffen hergestellt und anschließend in rechteckigen Formen ausgegossen.

**Tabelle 9**

| Zusammensetzung Schokolade - Mengenangaben als Gew.-% | |
|---|---|
| **Zubereitung** | **A** |
| Kakaomasse | 55,55 |
| Kakaobutter | 11,70 |
| Zucker | 29,50 |
| Magermilch | 3,00 |
| Lezithin | 0,2 |
| Vanillin | 0,035 |
| Homoeriodictyol 10 % in Ethanol | 0,05 |

### ANWENDUNGSBEISPIEL 8

### Zuckerfreie Hartkaramelle

Palatinit wurde mit Wasser gemischt und die Mischung bei 165 °C aufgeschmolzen und anschließend auf 115°C abgekühlt. Die übrigen Bestandteile wurden zugegeben und nach dem Durchmischen in Formen gegossen, nach dem Erstarren aus den Formen entfernt und anschließend einzeln verpackt.

**Tabelle 10**

| Zusammensetzung Karamelle - Mengenangaben als Gew.-% | | | |
|---|---|---|---|
| **Zubereitung** | **A** | **B** | **C** |
| Palatinit, Typ M | 75,00 | 74,00 | 75,00 |
| Zitronensäure | - | 1,0 | - |
| Wasser | 24,88 | 24,842 | 24,844 |
| Farbstoff gelb | - | 0,01 | - |
| Farbstoff blau | 0,01 | - | 0,01 |
| Pfefferminzaroma | 0,1 | - | 0,1 |
| Zitronenaroma | - | 0,1 | - |
| Rebaudiosid A 98 % | - | 0,040 | 0,040 |
| Hesperetin | - | 0,001 | 0,001 |
| Phloretin | - | 0,002 | - |
| Homoeriodictyol | 0,010 | 0,005 | 0,005 |
| Matairesinol | - | 0,005 | - |
| Enterolacton | - | - | 0,005 |

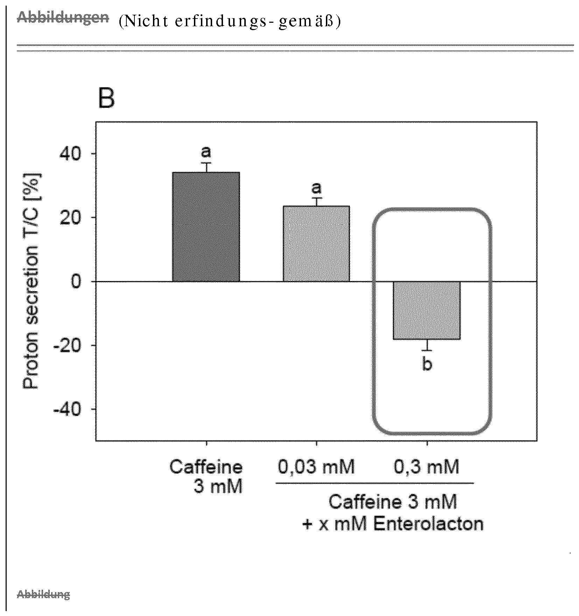

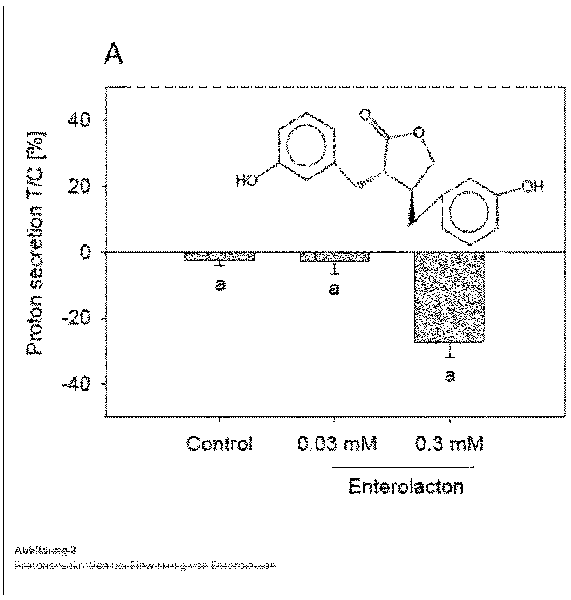

## Patentansprüche

1. Pharmazeutische Zubereitungen, enthaltend
bitter-maskierend wirkende Hydroxyflavanonen ausgewählt aus der Gruppe bestehend aus Eriodictyol-7-methylether, Homoeriodictyol, und deren Natrium-, Kalium-, Calcium-, Magnesium- oder Zinksalzen (Komponente a)
zur Verwendung zur Verminderung und/oder Inhibierung der von Nahrungsmittelbestandteilen (Komponente b) induzierten Magensäuresekretion,
ausgewählt aus der Gruppe, die gebildet wird von Xanthin, Fruchtsäuren, phenolischen Glykosiden, Flavonglykosiden, Dihydrochalconglykosiden, hydrolysierbaren und nicht-hydrolysierbaren Tanninen und deren Oligomeren, terpenoiden oder iridoiden Bitterstoffen und Triterpenglykosiden sowie deren Gemischen.

2. Zubereitungen zur Verwendung nach Anspruch 1, **dadurch gekennzeichnet, dass** die Magensäuresekretion induzierenden Nahrungsmittelbestandteilen ausgewählt sind aus der Gruppe, die gebildet wird von Coffein, Theobromin, Theophyllin, Weinsäure, Traubensäure, Äpfelsäure, Bernsteinsäure, Salicin, Arbutin, Neohesperidin, Eriocitrin, Neoeriocitrin, Narirutin, Naringin, Phloridzin, Trilobatin, Gallus- und Ellagsäureester von Kohlenhydraten, galloylierte Catechine und Epicatechine und deren Oligomere, Proanthocyanidine, Procyanidine, Thearubigenin, Quercetin, Quercitrin, Rutin, Taxifolin, Myricetin, Myricitrin, Limonin, Nomilin, Lupolone, Humolone, Stevioside, Rubodid, Steviosid, Rebaudiosid A, Rebaudiosid C, Glycyrrhizin, Glycyrrhetinsäure und Oleuropein sowie deren Gemische.

3. Pharmazeutische Zubereitungen zur Verwendung nach mindestens einem der Ansprüche 1 bis 2, **dadurch gekennzeichnet, dass** sie die Komponente (a) und (b) jeweils in Mengen von 0,1 bis 1.000 ppm enthalten.

4. Pharmazeutische Zubereitungen zur Verwendung nach mindestens einem der Ansprüche 1 bis 3, **dadurch gekennzeichnet, dass** sie die Komponente (a) und (b) jeweils in Mengen von 3 bis 300 ppm enthalten.

5. Zubereitungen zur oralen Aufnahme, enthaltend
(a) bitter-maskierend wirkende Hydroxyflavanonen ausgewählt aus der Gruppe bestehend aus Eriodictyol-7-methylether, Homoeriodictyol, und deren Natrium-, Kalium-, Calcium-, Magnesium- oder Zinksalzen (Komponente a), und
(b) Stoffe die die Sekretion von Magensäure induzieren, ausgewählt aus der Gruppe, die gebildet wird von Xanthin, Fruchtsäuren, phenolischen Glykosiden, Flavonglykosiden, Dihydrochalconglykosiden, hydrolysierbaren und nicht-hydrolysierbaren Tanninen und deren Oligomeren, terpenoiden oder iridoiden Bitterstoffen und Triterpenglykosiden sowie deren Gemischen (Komponente b),
**dadurch gekennzeichnet, dass** sie die Komponente (a) und (b) jeweils in Mengen von 3 bis 300 ppm enthalten.

6. Zubereitungen nach Anspruch 5, **dadurch gekennzeichnet, dass** es sich dabei um Backwaren, Süßwaren, alkoholische oder nicht-alkoholische Getränke, Fleischprodukte, Eier oder Eiprodukte, Getreideprodukte, Milchprodukte, Produkte aus Sojaprotein oder anderen Sojabohnen-Fraktionen, Fruchtzubereitungen, Gemüsezubereitungen, Knabberartikel, Produkte auf Fett- und Ölbasis oder Emulsionen derselben, sonstige Fertiggerichte und Suppen, Gewürze, Würzmischungen und Aufstreuwürzen handelt.

7. Zubereitungen nach Anspruch 5, **dadurch gekennzeichnet, dass** die die Magensäuresekretion induzierenden Nahrungsmittelbestandteile ausgewählt sind aus der Gruppe, die gebildet wird von Coffein, Theobromin, Theophyllin, Weinsäure, Traubensäure, Äpfelsäure, Bernsteinsäure, Salicin, Arbutin, Neohesperidin, Eriocitrin, Neoeriocitrin, Narirutin, Naringin, Phloridzin, Trilobatin, Gallus- und Ellagsäureester von Kohlenhydraten, galloylierte Catechine und Epicatechine und deren Oligomere, Proanthocyanidine, Procyanidine, Thearubigenin, Quercetin, Quercitrin, Rutin, Taxifolin, Myricetin, Myricitrin, Limonin, Nomilin, Lupolone, Humolone, Stevioside, Rubodid, Steviosid, Rebaudiosid A, Rebaudiosid C, Glycyrrhizin, Glycyrrhetinsäure und Oleuropein sowie deren Gemische.

## Claims

1. A pharmaceutical preparation, comprising
bitter-masking acting hydroxyflavones selected from the group consisting of eriodictiyol-7-methylether, homoeridictyol, and their sodium-, potassium-, calcium-, magnesium- or zinc salts (component a)
for use for reducing and/or inhibiting gastric acid secretion induced by food constituents (component b),
selected from the group consisting of xanthine, fruit acids, phenolic glycosides, flavone glycosides, dihydrochalcone glycosides, hydrolyzable and non-hydrolyzable tannins and oligomers thereof, terpenoid or iridoid bitter substances and triterpene glycosides and mixtures thereof.

2. Preparations for use according to claim 1, **characterized in that** said food constituents inducing gastric acid secretion are selected from the group consisting of caffeine, theobromine, theophylline, tartaric acid, racemic acid, malic acid, succinic acid, salicin, arbutin, neohesperidin, eriocitrin, neoeriocitrin, narirutin, naringin, phloridzin, trilobatin, gallic and ellagic esters of carbohydrates, galloylated catechins and epicatechins and oligomers thereof, proanthocyanidins, procyanidins, thearubigin, quercetin, quercitrin, rutin, taxifolin, myricetin, myricitrin, limonin, nomilin, lupolone, humolone, steviosides, rubodid, stevioside, rebaudioside A, rebaudioside C, glycyrrhizin, glycyrrhetic acid and oleuropein and mixtures thereof.

3. Pharmaceutical preparations for use according to at least one of claims 1 to 2, **characterized in that** said preparations comprise each of components (a) and (b) in amounts of from 0.1 to 1.000 ppm.

4. Pharmaceutical preparations for use according to at least one of claims 1 to 3, **characterized in that** said preparations comprise each of components (a) and (b) in amounts of from 3 to 300 ppm.

5. Preparations for oral consumption, comprising
(a) bitter-masking acting hydroxyflavones selected from the group consisting of eriodictiyol-7-methylether, homoeridictyol, and their sodium-, potassium-, calcium-, magnesium- or zinc salts (component a), and
(b) compounds, which induce gastric acid secretion, selected from the group consisting of xanthine, fruit acids, phenolic glycosides, flavone glycosides, dihydrochalcone glycosides, hydrolyzable and non-hydrolyzable tannins and oligomers thereof, terpenoid or iridoid bitter substances and triterpene glycosides and mixtures thereof (component b),
**characterized in that** said preparations comprise each of components (a) and (b) in amounts of from 3 to 300 ppm.

6. Preparation according to claim 5, **characterized in that** said preparation is in the form of bakery products, confectionery, alcoholic or non-alcoholic drinks, meat products, eggs or egg products, cereal products, milk products, products made from soy protein or other soybean fractions, fruit preparations, vegetable preparations, snack foods, products based on fat and oil or emulsions of the same, other ready meals and soups, spices, spice mixtures and seasonings.

7. Preparation according to claim 5, **characterized in that** said food constituents inducing gastric acid secretion are selected from the group consisting of caffeine, theobromine, theophylline, tartaric acid, racemic acid, malic acid, succinic acid, salicin, arbutin, neohesperidin, eriocitrin, neoeriocitrin, narirutin, naringin, phloridzin, trilobatin, gallic and ellagic esters of carbohydrates, galloylated catechins and epicatechins and oligomers thereof, proanthocyanidins, procyanidins, thearubigin, quercetin, quercitrin, rutin, taxifolin, myricetin, myricitrin, limonin, nomilin, lupolone, humolone, steviosides, rubodid, stevioside, rebaudioside A, rebaudioside C, glycyrrhizin, glycyrrhetic acid and oleuropein and mixtures thereof.

## Revendications

1. Préparations pharmaceutiques, contenant :
des hydroxyflavanones masquant l'amertume, choisies dans le groupe constitué par l'éther 7-méthylique d'ériodictyol, l'homo-ériodictyol et leurs sels de sodium, de potassium, de calcium, de magnésium ou de zinc (composant a),
destinées à une utilisation pour réduire et/ou inhiber la sécrétion d'acide gastrique induite par des constituants de produits alimentaires (composant b),
choisis dans le groupe qui est formé par la xanthine, les acides de fruits, les glycosides phénoliques, les glycosides de flavone, les glycosides de dihydrochalcone, les tannins hydrolysables et non hydrolysables et leurs oligomères, les substances amères terpénoïdes et iridoïdes et les glycosides de triterpènes, ainsi que leurs mélanges.

2. Préparations destinées à une utilisation selon la revendication 1, **caractérisées en ce que** les constituants de produits alimentaires induisant la sécrétion d'acide gastrique sont choisis dans le groupe qui est formé par la caféine, la théobromine, la théophylline, l'acide dioxysuccinique, l'acide tartrique, l'acide malique, l'acide succinique, la salicine, l'arbutine, la néohespéridine, l'ériocitrine, la néoériocitrine, la narirutine, la naringine, la phloridzine, la trilobatine, les esters d'acide gallique et ellagique de glucides, les catéchines et épicatéchines galloylées et leurs oligomères, les proanthocyanidines, les procyanidines, la théarubigénine, la quercétine, la quercitrine, la rutine, la taxifoline, la myricétine, la myricitrine, la limonine, la nomiline, la lupolone, l'humolone, les stéviosides, le rubodide, le stévioside, le rébaudioside A, le rébaudioside C, la glycyrrhizine, l'acide glycyrrhétique et l'oleuropéine, ainsi que leurs mélanges.

3. Préparations pharmaceutiques destinées à une utilisation selon au moins l'une quelconque des revendications 1 et 2, **caractérisées en ce qu'**elles contiennent les composants (a) et (b) à chaque fois en quantités de 0,1 à 1 000 ppm.

4. Préparations pharmaceutiques destinées à une utilisation selon au moins l'une quelconque des revendications 1 à 3, **caractérisées en ce qu'**elles contiennent les composants (a) et (b) à chaque fois en quantités de 3 à 300 ppm.

5. Préparations pour l'administration orale, contenant :
(a) des hydroxyflavanones masquant l'amertume, choisies dans le groupe constitué par l'éther 7-méthylique d'ériodictyol, l'homo-ériodictyol et leurs sels de sodium, de potassium, de calcium, de magnésium ou de zinc (composant a), et
(b) des substances qui induisent la sécrétion d'acide gastrique, choisies dans le groupe qui est formé par la xanthine, les acides de fruits, les glycosides phénoliques, les glycosides de flavone, les glycosides de dihydrochalcone, les tannins hydrolysables et non hydrolysables et leurs oligomères, les substances amères terpénoïdes et iridoïdes et les glycosides de triterpènes, ainsi que leurs mélanges (composant b),
**caractérisées en ce qu'**elles contiennent les composants (a) et (b) à chaque fois en quantités de 3 à 300 ppm.

6. Préparations selon la revendication 5, **caractérisées en ce qu'**il s'agit de produits de boulangerie, de confiseries, de boissons alcooliques ou non alcooliques, de produits à base de viande, d'oeufs ou de produits à base d'oeufs, de produits à base de céréales, de produits laitiers, de produits à base de protéine de soja ou d'autres fractions de soja, de préparations à base de fruits, de préparations à base de légumes, de produits à grignoter, de produits à base de matières grasses et d'huiles ou d'émulsions de ceux-ci, d'autres plats préparés et soupes, d'épices, de mélanges d'épices et d'épices en poudre.

7. Préparations selon la revendication 5, **caractérisées en ce que** les constituants de produits alimentaires induisant la sécrétion d'acide gastrique sont choisis dans le groupe qui est formé par la caféine, la théobromine, la théophylline, l'acide dioxysuccinique, l'acide tartrique, l'acide malique, l'acide succinique, la salicine, l'arbutine, la néohespéridine, l'ériocitrine, la néoériocitrine, la narirutine, la naringine, la phloridzine, la trilobatine, les esters d'acide gallique et ellagique de glucides, les catéchines et épicatéchines galloylées et leurs oligomères, les proanthocyanidines, les procyanidines, la théarubigénine, la quercétine, la quercitrine, la rutine, la taxifoline, la myricétine, la myricitrine, la limonine, la nomiline, la lupolone, l'humolone, les stéviosides, le rubodide, le stévioside, le rébaudioside A, le rébaudioside C, la glycyrrhizine, l'acide glycyrrhétique et l'oleuropéine, ainsi que leurs mélanges.
